# EUROPEAN PATENT APPLICATION

(11) **EP 2 236 615 A1**
(43) Date of publication of application: **06.10.2010**
(21) Application number: 08866334.9
(22) Date of filing: 12.12.2008
(51) Int. Cl.: C12Q 1/02, C12C 1/00, C12M 1/34, G01N 21/27

(54) **METHOD OF QUICKLY MEASURING FACTOR CAUSING EARLY FLOCCULATION OF YEAST AND A MEASUREMENT APPARATUS THEREFOR**

(30) Priority: 27.12.2007 JP 2007335976
(71) Applicant: KIRIN BEER KABUSHIKI KAISHA, Chuo-Ku Tokyo 104-8288 (JP)
(72) Inventor: TADA, Setsuzo, Tokyo 104-8288 (JP); NAKAHOSHI, Asumi, Tokyo 104-8288 (JP)
(74) Representative: HOFFMANN EITLE
(86) International application number: PCT/JP2008/072684
(87) International publication number: WO 2009/084407

(57) **Abstract**

This invention provides a method for rapidly measuring an premature yeast flocculating factor of malt contained in a brewing material, comprising the following steps of: (1) mixing yeast in the late logarithmic growth phase or thereafter with a water-extracted high molecular fraction prepared from a test material sample in a buffer solution and suspending the mixture in the buffer solution; and (2) irradiating the suspension obtained in the step (1) with visible light, photographing the scattered light with a camera device, image-analyzing the image data thus obtained and determining the degree of whiteness of the suspension to measure the degree of sedimentation of the yeast in the suspension. According to the present invention, the premature yeast flocculating factor of malt contained in the brewing material can be measured in a highly accurate, rapid and simple manner, and, further, even a plurality of analytes can be rapidly measured.

## Description

### CROSS-REFERENCES TO RELATED APPLICATIONS

This application is based upon and claims the benefit of priority from the prior Japanese Patent Applications No. 335976/2007, filed on December 27, 2007; the entire disclosures of which are incorporated herein by reference.

### BACKGROUND OF THE INVENTION

### Field of Invention

The present invention relates to a method for rapidly measuring an premature yeast flocculating factor of malt contained in a brewing material for use in the production of, for example, fermented malt beverages such as beers, sparkling wines, and whiskies, and a method for rapidly determining an premature yeast flocculating property of malt in a brewing material using the method. The present invention also relates to an apparatus for continuously and quantitatively measuring the sedimentation of cells in a suspension.

### Background Art

In brewing of, for example, fermented malt beverages such as beers, sparkling wines, and whiskies, the yeast moderately flocculates and settles out at the end of main fermentation with yeast. The flocculating sedimentation makes it possible to collect the yeast. When the yeast does not moderately flocculate and settle out at the end of main fermentation, the amount of the yeast collected is unsatisfactory. On the other hand, when the yeast excessively flocculates and settles out at the end of main fermentation, disadvantageously, the fermentation does not proceed. The flocculation of the yeast occurs as a result of the formation of yeast mass at the end of fermentation where the amount of extract is small. The flocculation causes the yeast to settle out at the bottom from a culture solution. There is a report, for example, about the participation of bonding between lectin-like proteins on the surface layer of yeast cells and mannose sugar chains in yeast mannan in the flocculation of the yeast per se (Appl. Microbiol. Biotechno 123: 197-205, 2003).

In the brewing of, for example, fermented malt beverages, in a normal fermentation process, the yeast moderately flocculates and settles out at the end of main fermentation with yeast where the amount of the extract of the fermentation liquor is small. Regarding the flocculating sedimentation of the yeast, it has been reported that a phenomenon called "premature yeast flocculation phenomenon of malt" (hereinafter sometimes referred to as "premature yeast flocculation phenomenon") is observed.

The "premature yeast flocculation phenomenon" refers to a phenomenon that, despite the fact that, in a fermentation process using yeast, particularly at late phase of fermentation, assimilable sugar of the yeast is still present in the fermentation liquor, the yeast disadvantageously flocculates and settles out. When the yeast flocculates and settles out by the premature yeast flocculation phenomenon, the fermentation no longer proceeds (Proc. Congr. Eur. Brew. Conv. 26: 53-60, 1997). Therefore, when this phenomenon occurs, the fermentation is unsatisfactory and, as a result, the obtained product is below the standard, leading to a large loss in brewing of fermented malt beverages and the like.

It is considered that the premature yeast flocculation phenomenon is derived from raw material wheat or barley and is caused by high molecular acid polysaccharides contained in the malt (J. Am. Soc. Brew. Chem. 47: 29-34, 1989). It is known that factors causative of the premature yeast flocculation phenomenon are produced in a malting process or are inherently present in the raw material wheat or barley (J. Inst. Brew., 97, 359-366, 1991; Japanese Patent Application Laid-Open Publication No. 179190/1998). In brewing of, for example, fermented malt beverages using barley as a raw material, from the viewpoint of avoiding the premature yeast flocculation phenomenon in the fermentation process, it has hitherto been desired to adopt a method in which whether or not malt or barley used has premature yeast flocculating properties is confirmed and malted barley which do not cause the premature yeast flocculation phenomenon is selected and used.

In order to confirm whether or not malt, barley and the like have premature yeast flocculating properties, methods using a fermentation test has hitherto been adopted. Examples of such methods include a KY tube fermentation test method developed by T. Nakamura et al. (Proc. Congr. Eur. Brew. Conv. 26: 53-60, 1997) and a universal fermentation test developed by M. Jibiki et al. (J. Am. Soc. Brew. Chem. 64(2): 79-85, 2006). In both the methods, however, the fermentation test is carried out on a scale reduced from an actual brewing scale (a fermentation test scale), and, thus, wort should be fermented. Further, in these methods, one day for preparing wort and about eight days for confirming the presence or absence of an premature yeast flocculating factor in the malt or barley from the progress of fermentation are generally necessary. Further, for wheat and barley before malting, about 7 days are necessary for malting and the preparation of wort, and, thus, a long period of time is necessary for confirming the presence or absence of the premature yeast flocculating factor.

In order to shorten the fermentation test period for confirming whether or not the malt, barley or wheat has premature yeast flocculating properties, a method has been developed that comprises adding enzyme to a test raw material malt, barley or wheat, enzymatically treating the raw material malt, barley or wheat, adding the enzyme treated product or a high molecular fraction separated from the enzyme treated product to synthetic wort to prepare a fermentation test material, and, 48 hr after the start of the test, measuring the turbidity of the fermentation test material to determine whether or not premature yeast flocculating factors are present in the raw material malt, barley or wheat (Japanese Patent Application Laid-Open Publication No. 179190/1998). According to this method, the period of time necessary for confirming the presence or absence of premature yeast flocculating factors could be significantly shortened. This method, however, also utilizes a fermentation test, and, thus, a long period of time of 48 hr is still necessary for the fermentation test.

International Publication WO 2005/073394 A1. discloses a method called a cuvette method. This method comprises mixing premature yeast flocculating factors extracted from malt with yeast in the late logarithmic growth phase in a cuvette and measuring flocculation and sedimentation of yeast based on absorbance at a specific wavelength. Therefore, this method does not require the fermentation test. This method, however, is carried out by a manual analysis and thus is not suitable for multianalyte treatment and provides room for the occurrence of problems with the accuracy of treatment and measuring errors caused by a difference in person who performs the analysis.

Accordingly, the development of a method that, in brewing of fermented malt beverages and the like, can determine the presence or absence of premature yeast flocculating factors in a brewing material in a simpler manner in a shorter period of time and further can realize a multianalyte analysis in a rapid and efficient manner has been still desired.

### SUMMARY OF THE INVENTION

The present inventors have now found that the measurement of premature yeast flocculating factors of malt contained in a raw material for producing fermented malt beverages in a very short time and simultaneous measurement of a plurality of analytes can be realized by mixing yeast in the late logarithmic growth phase or thereafter with a water-extracted high molecular fraction prepared from a test material sample such as malt, barley or wheat or malt in a buffer solution, suspending the mixture in the buffer solution, and measuring the degree of sedimentation of the yeast suspended in the suspension in such a manner that the suspension is irradiated with visible light, the scattered light is photographed with a digital video camera, and the image data thus obtained is subjected to an image analysis to digitize the data as the degree of whiteness. The present inventors have further found that the efficiency of the whole measuring treatment can be significantly improved by simultaneously performing the mixing and suspending treatment for a plurality of analytes and, consequently, the plurality of analytes can be automatically measured with minimized manpower. Further, the present inventors have succeeded, based on such studies, developing an apparatus for continuously and quantitatively measuring the sedimentation of cells in the suspension. The present invention has been made based on such finding.

Accordingly, an object of the present invention is to provide a method that can measure premature yeast flocculating factors of malt contained in a brewing material in a highly accurate, rapid and simple manner and further can rapidly measure a plurality of analytes.

According to the present invention, there is provided a method for rapidly measuring an premature yeast flocculating factor of malt contained in a brewing material, comprising the following steps of:
(1) mixing yeast in the late logarithmic growth phase or thereafter with a water-extracted high molecular fraction prepared from a test material sample in a buffer solution to suspend the mixture of the yeast and the water-extracted high molecular fraction in the buffer solution; and
(2) irradiating the suspension obtained in the step (1) with visible light, photographing the scattered light with a camera device, image-analyzing the image data thus obtained and determining the degree of whiteness of the suspension to measure the degree of sedimentation of the yeast in the suspension.

In the method according to the present invention, preferably, the step (2) comprises photographing the scattered light with a digital camera device and subjecting the image data thus obtained to an image analysis to determine the digitized degree of whiteness of the suspension.
According to another preferred embodiment of the present invention, the method according to the present invention comprises digitizing the degree of whiteness of the suspension by presuming the degree of whiteness at lights-out and the degree of whiteness of the suspension in which the yeast immediately after the mixing and suspending is homogeneously dispersed to be 0 and 100, respectively.

According to still another one preferred embodiment of the present invention, in the step (2), the irradiation of the suspension with the visible light is carried out from a light source placed just under the suspension, and the light scattered in the suspension is photographed with a camera device installed in a direction horizontal to the suspension.

According to a further one preferred embodiment of the present invention, in the step (1), a plurality of analytes are subjected to simultaneous mixing and suspending in a multianalyte shaking device which allows a plurality of analytes to undergo simultaneous shaking, thereby obtaining a plurality of suspensions. In this case, more preferably, the degrees of sedimentation of yeasts in a plurality of respective suspensions are simultaneously measured.

According to another one preferred embodiment of the present invention, the yeast used in the step (1) is one obtained by cultivating yeast and harvesting yeast in the late logarithmic growth phase or thereafter, or one obtained by further cryopreserving the harvested yeast. In this case, more preferably, the harvested yeast has been washed with EDTA.

According to still another one preferred embodiment of the present invention, the high molecular fraction used in the step (1) is a high molecular fraction prepared by subjecting the water extract of the test material sample to ethanol precipitation, or a high molecular fraction obtained by separating the water extract of the test material sample by dialysis, ultrafiltration, or gel filtration.
According to a further one preferred embodiment of the present invention, the high molecular fraction used in the step (1) is prepared from a saccharified liquid of the test material sample.
According to another one preferred embodiment of the present invention, in preparing the water-extracted high molecular fraction used in the step (1), the test material sample is enzymatically treated during the extraction.

According to still another one preferred embodiment of the present invention, the buffer solution used in the step (1) is acetate buffer-CaCl₂.

According to a further one preferred embodiment of the present invention, the buffer solution used in the step (1) is acetate buffer-CaCl₂ to which a saccharide component selected from the group consisting of glucose, maltose, mannose, and their mixtures has been added.

According to one preferred embodiment of the present invention, the test material sample is barley, malt, or barley in the course of malting.
According to another one preferred embodiment of the present invention, the water-extracted high molecular fraction in the test material sample is a high molecular fraction of an extract obtained by extracting a ground product of malt with water for 30 sec or more, or a high molecular fraction of an extract obtained by extracting a ground product of barley or a ground product of barley in the course of malting with water for 15 min or more.

According to the present invention, there is provided a method for rapidly determining an premature yeast flocculating property in a brewing material, **characterized in that** the method utilizes the method for rapidly measuring an premature yeast flocculating factor of malt according to the present invention. This method typically comprises the step of determining, based on the results obtained by the method for rapidly measuring an premature yeast flocculating factor of malt according to the present invention, whether or not a brewing material has an premature yeast flocculating property.

Further, according to the present invention, there is provided a process for producing malt, characterized by comprising determining an premature yeast flocculating property of a malt raw material, in-process malt, or produced malt by the method for rapidly measuring an premature yeast flocculating factor of malt according to the present invention to manage a malt production process.

Furthermore, according to the present invention, there is provided a process for producing a fermentative alcohol beverage, characterized by comprising determining an premature yeast flocculating property of a brewing material by the method for rapidly measuring an premature yeast flocculating factor of malt according to the present invention to select and regulate the brewing material used.

Furthermore, according to the present invention, there is provided an apparatus for continuously and quantitatively measuring the sedimentation of cells in a suspension, the apparatus comprising:
analyte shaking means that horizontally holds a plurality of cuvettes or vials, in which a suspension as an analyte is to be placed, at a predetermined position in a line, and if necessary shakes the cuvettes or vials to suspend the suspension contained in the cuvettes or vials;
a camera device that photographs the cuvettes or vials disposed in a line; and
data processing means that subjects image data photographed with the camera device to an image analysis to obtain digitized data on the degree of whiteness of the suspensions.

According to a preferred embodiment of the present invention, the analyte shaking means has a visible light source just under the cuvettes or vials.

According to another one preferred embodiment of the present invention, the apparatus further comprises control means that controls the analyte shaking means and the camera device so that, after shaking of the cuvettes or vials for a predetermined period of time in the analyte shaking means, the cuvettes or vials are allowed to stand for a predetermined period of time and are photographed with the camera device.

According to still another one preferred embodiment of the present invention, the camera device in the measuring apparatus is a digital video camera.

According to a preferred embodiment of the present invention, the apparatus is used in rapid measurement of an premature yeast flocculating factor of malt contained in a brewing material.

According to a more preferred embodiment of the present invention, the apparatus carries out the method for rapidly measuring an premature yeast flocculating factor of malt according to the present invention.

The method for rapidly measuring an premature yeast flocculating factor of malt according to the present invention is advantageous in that, in brewing of fermented malt beverages and the like, the presence or absence of an premature yeast flocculating factor in a brewing material can be determined in a simple, rapid and accurate manner without relying upon the conventional fermentation method and that, unlike the conventional cuvette method that uses absorbance, a plurality of analytes can be simultaneously measured in an accurate and efficient manner. In the cuvette method, how to mix yeast with a water-extracted high molecular fraction is not clearly specified. Accordingly, there is a possibility that persons responsible for the analysis performs the mixing by mutually different methods, leading to room for the occurrence of problems with the accuracy of the treatment and measuring errors. By contrast, in the method according to the present invention, the treatment is simple, and a plurality of analytes can be simultaneously measured. Therefore, there is no such possibility involved in the cuvette method. Further, according to the method of the present invention, the plurality of analytes can be automatically measured with minimized manpower. Thus, the method according to the present invention is very useful as a practical method that can measure and determine an premature yeast flocculating property of a brewing material used in brewing of fermented malt beverages and the like. In particular, the rapid, efficient and accurate measurement of a plurality of analytes leads to great expectations of the application of the method according to the present invention to the production of fermented malt beverages and the like on a commercial scale. This is true of the apparatus according to the present invention.

The method for rapidly measuring an premature yeast flocculating factor of malt according to the present invention can be applied to the measurement of an premature yeast flocculating factor in malt or barley before malting, as well as barley immediately after harvesting, barley during malting, or other grain and extracts or the like used in brewing and can be widely used as a simple, rapid and reliable method in the determination of whether or not an premature yeast flocculating factor is present in these brewing materials. Further, the measuring method according to the present invention can realize measurement using only a small amount of a sample and thus can easily be used in brewing of fermented malt beverages or the like and can be provided as a highly accurate practical measuring and determining method. Further, the method for measuring an premature yeast flocculating factor according to the present invention can accurately figure out the measurement of an premature yeast flocculating factor for each raw material and thus can determine the premature yeast flocculating property for each raw material. Further, the measuring method according to the present invention can also measure the activity of a very small amount of an premature yeast flocculating factor and thus can measure an premature yeast flocculating factor for each of divided fractions in each of brewing materials and can be effectively utilized for the purification and identification of the premature yeast flocculating factor.

### BRIEF DESCRIPTION OF DRAWINGS

[Fig. 1] Fig. 1 is a conceptual view showing the positional relationship among a camera device, a cuvette or a vial, and a light source.
[Fig. 2] Fig. 2 is a conceptual view showing an apparatus according to the present invention.
[Fig. 3] Fig. 3 is an image 10 min after the start of monitoring in Example 1.
[Fig. 4] Fig. 4 is a graph showing the results of measurement in Example 2.
[Fig. 5] Fig. 5 is a conceptual view showing how to divide vials into five stages for the measurement of turbidity.
[Fig. 6] Fig. 6 is a graph showing the results of measurement in Example 3.

### DETAILED DESCRIPTION OF THE INVENTION

### Method for rapidly measuring premature yeast flocculating factor of malt

As described above, the measuring method according to the present invention is a method for rapidly measuring an premature yeast flocculating factor of malt contained in a brewing material, comprising the following steps of:
(1) mixing yeast in the late logarithmic growth phase or thereafter with a water-extracted high molecular fraction prepared from a test material sample in a buffer solution to suspend the mixture of the yeast and the water-extracted high molecular fraction in the buffer solution; and
(2) irradiating the suspension obtained in the step (1) with visible light, photographing the scattered light with a camera device, image-analyzing the image data thus obtained and determining the degree of whiteness of the suspension to measure the degree of sedimentation of the yeast in the suspension.

In the present invention, the brewing material refers to those used in the production of, for example, fermented malt beverages such as beers, sparkling wines, and whiskies, and examples of such brewing materials include malt or barley before malting, as well as barley immediately after harvesting, barley during malting, or other grain and extracts or the like used in brewing. The same materials may be mentioned as the test material sample. The test material sample is preferably barley, malt, or barley during malting.

### Step (1):

In the step (1) in the measuring method according to the present invention, yeast in the late logarithmic growth phase or thereafter and a water-extracted high molecular fraction prepared from a test material sample are mixed together in and suspended in a buffer solution.
In the present invention, as with the cuvette method as described in International Publication WO 2005/073394A1, "yeast in the late logarithmic growth phase or thereafter" is used in the measurement of an premature yeast flocculating factor of malt. The reason why the "yeast in the late logarithmic growth phase or thereafter" is used is that, at this phase, the yeast begins to acquire a moderate flocculating capability.

The "yeast in the late logarithmic growth phase or thereafter" can be prepared, for example, by the following method. Yeast for brewing is cultivated in a commonly used medium for yeast, a growth curve for the yeast is prepared, and yeast which has reached the late logarithmic growth phase or thereafter (usually yeast on day 4 or day 5 from the start of cultivation at a temperature of 8°C) is separated and harvested by centrifugation or the like. The harvested yeast is preferably washed, for example, with a chelate compound solution such as 0.1 M EDTA. The harvested yeast as such may be used. Alternatively, the cultivated and harvested yeast may be cryopreservated, for example, using a 15% glycerol solution at -80°C. The cryopreservated yeast may be, for example, thawed and then used in the measuring method according to the present invention.

The "water-extracted high molecular fraction prepared from a test material sample" used in the measuring method according to the present invention can be prepared, for example, by the following method.
At the outset, a test material sample such as barley or malt is if necessary ground and is extracted with water or undergoes conventional saccharification. In extracting the test material sample with water, the test material sample can be enzymatically treated by the addition of α-amylase, β-amylase, β-glucanase, protease or the like according to the method described in Japanese Patent Application Laid-Open No. 179190/1998. In preparing the water-extracted high molecular fraction of the test material sample, the high molecular fraction can be separated and recovered from the water extract, for example, by an ethanol precipitation method, for example, in which ethanol is added to the water extract to a final concentration of 50% and the resultant precipitate is collected by centrifugation, or by fractionation and recover, for example, using dialysis, ultrafiltration, or gel filtration.

In the step (1) in the measuring method according to the present invention, the yeast and the water-extracted high molecular fraction are mixed together in and suspended in a buffer solution. The buffer solution used herein is preferably acetate buffer-CaCl₂. More preferably, the buffer solution is, for example, a liquid of 50 mM acetate buffer-(pH 4 to 4.5)-0.1% CaCl₂.

Here, for example, yeast harvested in the late stationary phase of the growth and yeast harvested in plant and the like per se sometimes have a high flocculating properly. When the yeast used has the high flocculating property, there is a possibility that, even when the measurement is carried out using normal malt (non-premature yeast flocculating malt), the yeast settles out and, consequently, the non-premature yeast flocculating property of the raw material cannot be accurately measured. For this reason, preferably, a measurement noise derived from the highly flocculating yeast is eliminated by suppressing flocculation derived from yeast which per se has a high flocculating property while substantially avoiding an influence on the premature yeast flocculating factor-derived flocculation. The present inventors have further succeeded in properly suppressing a flocculation noise derived from the undesired yeast by using a buffer solution prepared by adding a succharide component (for example, monosaccharide, disaccharide, or their mixture) to acetate buffer-CaCl₂ (Example 4). It is considered that the saccharide component is effective in inhibiting bonding between the yeast and the premature yeast flocculating factor.

Thus, according to another one preferred embodiment of the present invention, the buffer solution is a solution prepared by adding a saccharide component (for example, monosaccharide, disaccharide, or their mixture) to acetate buffer-CaCl₂. The saccharide component to be added is preferably selected from the group consisting of glucose, maltose, mannose, and their mixtures.

When the saccharide component added is maltose, the concentration of maltose in the buffer is preferably 0.5 to 1.5% by weight, more preferably 0.8 to 1.2% by weight, particularly preferably about 1% by weight. It is known that the level of the effect of inhibiting bonding between the yeast and the premature yeast flocculating factor by the saccharide component varies depending upon the type of the saccharide. In the case of the above-described saccharides, the level of the inhibitory effect is known to be mannose > glucose > maltose. Accordingly, when mannose or glucose having a higher inhibitory effect than maltose is used, the concentration of mannose or glucose used may be lower than the concentration of maltose.

According to a particularly preferred embodiment of the present invention, the buffer solution used is a liquid of 50 mM acetate buffer-(pH 4 to 4.5)-0.1% CaCl₂-1% maltose.

Further, as described above, in the present invention, preferably, the addition of the saccharide component to the buffer solution is adopted when the yeast used has a high flocculating property. Whether or not the yeast used has a high flocculating properly can easily be confirmed by examining whether or not the yeast settles out even when normal malt is used.

The mixing and suspending treatment is carried out by a conventional method in which a vessel such as a cuvette or a vial containing a suspension (a mixture of the yeast with the water-extracted high molecular fraction) as an analyte undergoes stirring or shaking. In the present invention, preferably, a plurality of analytes are subjected to simultaneous mixing and suspending in a multianalyte shaking device which allows a plurality of analytes to undergo simultaneous shaking, thereby obtaining a plurality of suspensions. This is useful for simultaneous measurement of a plurality of analytes, As described later, preferably, the multianalyte shaking device horizontally holds a plurality of cuvettes or vials, in which a suspension as an analyte is to be placed, at a predetermined position in a line, and if necessary shakes the cuvettes or vials to suspend the suspension contained in the cuvettes or vials.

### Step (2):

The step (2) in the measuring method according to the present invention comprises irradiating the suspension obtained in the step (1) with visible light, photographing the scattered light with a camera device, image-analyzing the image data thus obtained and determining the degree of whiteness of the suspension to measure the degree of sedimentation of the yeast in the suspension.

In the conventional cuvette method, the degree of sedimentation of yeast in the suspension is determined by measuring the optical density of the suspension with a spectrophotometer. On the other hand, according to the present invention, light scattered as a result of irradiation of the suspension with visible light is photographed with a camera device, and the image date thus obtained is subjected to an image analysis. Accordingly, unlike the conventional method in which analytes are manually analyzed in order one by one, in the present invention, image data can be obtained by arranging a plurality of analytes and simultaneously photographing scattered light for the plurality of analytes with the camera device. According to the present invention, a plurality of analytes can be simultaneously measured in a short time by subjecting the image data to an image analysis.
Further, according to the present invention, since photographing and image analysis can be continuously carried out, the measuring method according to the present invention can be continuously carried out and, thus, for example, a time-dependent change can also be measured.

Any light source may be used for the application of visible light to the suspension as long as light scattered by applying light from the light source to the suspension can be photographed with a camera device which will be described later and, further, the photographed image data can be subjected to an image analysis to digitize the degree of whiteness. For example, a commercially available white LED light source can be used as the light source. Likewise, the luminous intensity of the light source is not limited as long as the measurement according to the present invention can be carried out. However, light having a luminous intensity of approximately 10000 to 30000 mcd, for example, approximately 18000 mcd, may be applied to the analyte.

The camera device used is not particularly limited as long as the photographed image data can be output to an image processing device. Accordingly, in general, the camera device is preferably a digital camera, more preferably a digital video camera. The number of pixels of the digital camera is not particularly limited as long as the image analysis can be carried out based on the image data to determine the degree of whiteness. Preferably, however, the number of pixels is, for example, 1.3 million or more.

Thus, according to a preferred embodiment of the present invention, in the step (2), the scattered light is photographed with a digital camera device, and the image data thus obtained are image-analyzed to determine the digitized degree of whiteness of the suspension.

The digitized degree of whiteness can be obtained, for example, by subjecting the obtained image data to an image analysis to determine, as the degree of whiteness, the ratio between white and black in the region to be measured. Preferably, the degree of whiteness of the suspension is digitized as relative values by presuming the degree of whiteness at lights-out and the degree of whiteness of the suspension in which the yeast immediately after the mixing and suspending is homogeneously dispersed to be 0 and 100, respectively. In this case, as the degree of whiteness increases, the numerical value increases between 0 and 100.

The image analysis of the image data can be carried out by combining a personal computer with a commercially available software that can digitize areas of white and black in the image.

According to a preferred embodiment of the present invention, the irradiation of the suspension with the visible light is carried out from a light source placed just under the suspension. In this case, the light scattered in the suspension is generally photographed with a camera device installed in a direction horizontal to the suspension. According to this constitution, light scattered by the suspended yeast can be accurately captured. Further, problems with treatment accuracy and measurement errors derived from a difference in person who performs the analysis can be significantly reduced.

According to one preferred embodiment of the present invention, after mixing and suspending in the step (1), the suspension is optionally allowed to stand for a predetermined period of time, followed by resuspending and the measurement of the degree of sedimentation of yeast in the suspension. The standing time is not particularly limited as long as yeast mixed into the suspension can be reacted with an premature yeast flocculating factor to form flocculates.
Further, the time of suspension after standing is not particularly limited as long as the yeast flocculates in the suspension can be brought to a homogeneously dispersed state. Thus, the provision of given standing time and suspending time can provide a distinct degree of sedimentation of yeast and can realize accurate and highly reproducible measurement with little or no variation.

In the measuring method according to the present invention, as described above, the degree of sedimentation of yeast in the suspension is measured by determining the degree of whiteness of the suspension. That is, as the degree of sedimentation of yeast increases, the suspension when observed from its side is viewed as deeper black and, thus, the degree of whiteness decreases. On the other hand, as the degree of sedimentation decreases, that is, the amount of yeast which has settled out decreases, the suspension is viewed as white and the degree of whiteness increases. Thus, the degree of sedimentation of yeast can be determined based on the degree of whiteness.

According to a more preferred embodiment of the present invention, the degrees of sedimentation of yeasts in a plurality of respective suspensions are simultaneously measured. In this case, more preferably, a plurality of analytes are simultaneously subjected to mixing and suspending treatment in the step (1). Thus, when a plurality of analytes can be simultaneously subjected to the step (1) and the step (2), the measuring method according to the present invention can be automated without relying upon manpower.

### Measuring apparatus

According to the present invention, as described above, there is provided an apparatus for continuously and quantitatively measuring the sedimentation of cells in a suspension, the apparatus comprising:
analyte shaking means that horizontally holds a plurality of cuvettes or vials, in which a suspension as an analyte is to be placed, at a predetermined position in a line, and if necessary shakes the cuvettes or vials to suspend the suspension contained in the cuvettes or vials;
a camera device that photographs the cuvettes or vials disposed in a line; and
data processing means that subjects image data photographed with the camera device to an image analysis to obtain digitized data on the degree of whiteness of the suspensions.

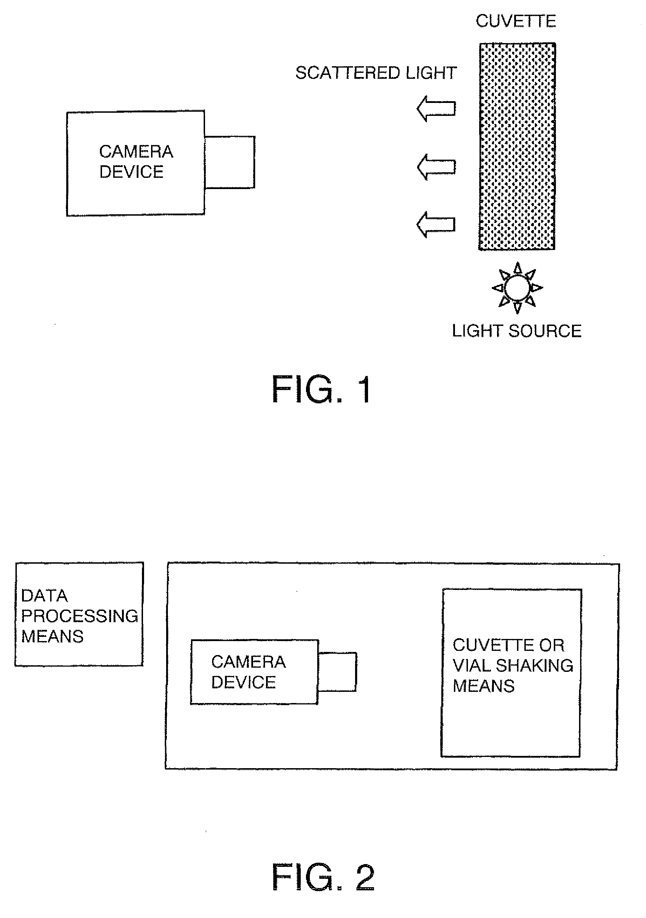

Fig. 1 is a conceptual view showing the positional relationship among a camera device, a cuvette or a vial, and a light source. Fig. 2 is a conceptual view showing an apparatus according to the present invention.

Here cells are, for example, yeast or fungal cells, preferably yeast cells. The measuring method according to the present invention can also be applied to living organisms in general that can flocculate and settle out.

The analyte shaking means can horizontally hold a plurality of cuvettes or vials, in which a suspension as an analyte is to be placed, at a predetermined position in a line, and if necessary can shake the cuvettes or vials to suspend the suspension contained in the cuvettes or vials.

The expression "a plurality (for example, 11 or 12) cuvettes or vials are horizontally held at a predetermined position in a line" means that a plurality of cuvettes or vials are held at such height and position (position relative to a camera device) that the plurality of cuvettes or vials can be simultaneously photographed with a camera device and that the plurality of cuvettes or vials are disposed in a line generally at a right angle to a direction in which photographing with the camera device is carried out so that the plurality of cuvettes or vials can be simultaneously photographed with the camera device. The position of the cuvettes or vials can be properly set as long as the above requirement is met. Further, in this case, the height, position and arrangement of the cuvettes or vials and the positional relationship between the cuvettes and the camera device can be properly changed as long as the plurality of analytes can be simultaneously photographed with the camera device.

The analyte shaking means is not particularly limited as long as the analyte shaking means is a device that can if necessary shake cuvettes or vials held at a predetermined position to perform suspending of the suspension contained in the cuvettes or vials. For example, a multianalyte shaking device which can fix a plurality of cuvettes or vials in parallel (in a line) and can shake the cuvettes or vials while reversing the cuvettes or vials. Preferably, in the multianalyte shaking device, the shaking speed, shaking time, and waiting time are variable and can be properly set. The shaking speed, shaking time, and waiting time can be controlled with a separate control unit. Close control of the shaking speed, shaking time, and waiting time by the control unit or the like can eliminate measurement errors derived from a difference in person who performs the analysis. For example, a commercially available product may also be used as the control unit.

More preferably, the apparatus according to the present invention further comprises control means that controls the analyte shaking means and the camera device so that, after shaking of the cuvettes or vials for a predetermined period of time in the analyte shaking means, the cuvettes or vials are allowed to stand for a predetermined period of time and are photographed with the camera device. Thus, controlling the operation of mixing, suspending, standing, and photographing with a camera can realize the automation of the measurement and thus can substantially eliminate measurement errors that may occur due to a difference in person who performs the analysis. Further, better measurement conditions can be found and can be set.
For example, for glass vials, mixing for 2 min → standing (still standing) for 15 min or longer → mixing for 2 min → measurement (photographing) may be mentioned as an example of conditions for such control. Further, for cuvettes, mixing for 7 min → standing (still standing) for 2 min → mixing for 7 min → measurement (photographing) may be mentioned as an example of conditions for such control.

According to a preferred embodiment of the present invention, the analyte shaking means has a visible light source disposed just under the cuvettes or vials.

In the present invention, the data processing means can perform an image analysis of the image data photographed with the camera device to obtain digitized data on the degree of whiteness of the suspensions. For example, a personal computer may be mentioned as the processing means. In this case, preferably, the personal computer is used in combination with a software that can perform an image analysis of the obtained image data, for example, a commercially available software that can digitize areas of white and block in the images.

In the present specification, the expressions of values using "about" and "approximately" means that, in attaining an object of setting of the value, a variation in value which can be accepted by a person having ordinary skill in the art is included. For example, the expressions of values using "about" and "approximately" means that a variation within not more than 20%, preferably within not more than 10%, more preferably within not more than 5%, of a predetermined value or range is acceptable.

### EXAMPLES

The present invention is further illustrated by the following Examples that are not intended as a limitation of the invention.

### Experimental method

### 1) Cultivation of yeast

A preculture solution produced by stationary cultivation in a YEPG medium (1% yeast extract, 2% bactopepton, 7.5% maltose, and 2.5% glucose) at 20°C for 3 days was added at a concentration of 1.5% to a YEPG medium (usually 100 ml of the medium in a 250 ml-volume medium bottle) which had been previously cooled to 8°C, and the mixture was cultivated while stirring with a stirrer at 8°C. During the cultivation, OD600 was measured over time, and a growth curve for yeast was prepared so that yeast at a logarithmic growth phase, yeast at a late logarithmic growth phase where the growth curve begins to become flat (on day 4 after the start of the cultivation), and yeast at an early stationary phase (on day 5, 10 hr after the start of the cultivation) could be used.

### 2) Preparation of yeast for test

Yeast at an early stationary phase after the completion of the cultivation was collected by centrifugation at 3000 rpm for 5 min (4°C). The collected yeast was washed by suspending in cold water, and the yeast was collected in the same manner as described just above, was washed twice by suspending in cooled 100 mM EDTA (pH 8.0), was washed twice by suspending in cold water, and was suspended in 20 ml of cold water. The yeast liquid thus obtained was diluted to a 1 : 200 concentration, and OD600 of the diluted yeast liquid was measured to determine the concentration of yeast. Further, the yeast liquid was washed twice with cold water and was suspended in a 15% glycerol liquid, and OD600 of the yeast liquid was measured in the same manner as described above to determine the concentration of yeast, followed by lyophilization at -80°C.

### 3) Preparation of water-extracted high molecular fraction of malt

Water (120 ml) was added to 20 g of each of the following premature yeast flocculating malts and non-premature yeast flocculating malts (normal malts), and each of the mixtures was stirred with a stirrer for 10 min. The stirred mixtures were then centrifuged at a temperature of 4°C at 8718 g for 10 min to remove an unnecessary fraction and to collect supernatants. The supernatants were further filtered to remove insoluble fractions, and the filtrates were admixed with an equal amount of ethanol. The admixtures were allowed to stand at room temperature for 10 min and were then centrifuged at a temperature of 4°C at 11387 g for 10 min to collect precipitate fractions. The precipitate fractions were suspended in 10 ml of hot water to give measuring samples.
Premature yeast flocculating malts 1 to 3: samples for evaluation of malts, manufactured by Kirin Brewery Company, Limited
Normal malts 1 to 3: samples for evaluation of malts, manufactured by Kirin Brewery Company, Limited

### Example 1: Identification of premature yeast flocculating malt

Water was added to the lyophilized yeast obtained in 2) to a predetermined concentration (OD600: about 200) and thus to give a yeast liquid. On the other hand, the water-extracted high molecular fractions of malt (premature yeast flocculating malts 1 to 3 and normal malts 1 to 3) obtained in 3) were provided.

Subsequently, 0.075 ml of the yeast liquid and 0.385 ml of the water-extracted high molecular fraction of malt were suspended in 1.5 ml of 50 mM sodium acetate (pH 4.8) and 6 µl of 50% calcium chloride, and the mixture was adjusted to a total volume of 3 ml by the addition of water. Each of the solutions thus obtained was placed in a plastic cuvette (10 × 10 × 45 mm), and the top of the cuvette was sealed with Parafilm.

The cuvettes were placed on a holder within a multianalyte shaking device and were subjected to a series of procedures: shaking for 7 min → standing for 2 min → shaking for 7 min. Thereafter, sedimentation were monitored under the following conditions.

The multianalyte shaking device used comprises a shaking device part that can fix 11 cuvettes in parallel and can shake the cuvettes while inverting the cuvettes.
A white LED light source (NSPW500BS, manufactured by NICHIA Corporation) (AC100V/3A) was used as a light source.
KV-16AT manufactured by KEYENCE CORPORATION was used as a control unit for the shaking device.
A digital video camera (ARTCAM-130MI (1.5 million pixels), manufactured by ARTRAY CO., LTD.) was used as a camera device.
A commercially available computer (OS: WindoowsXP) was used as a personal computer, and a commercially available sedimentation rate analysis software was used,

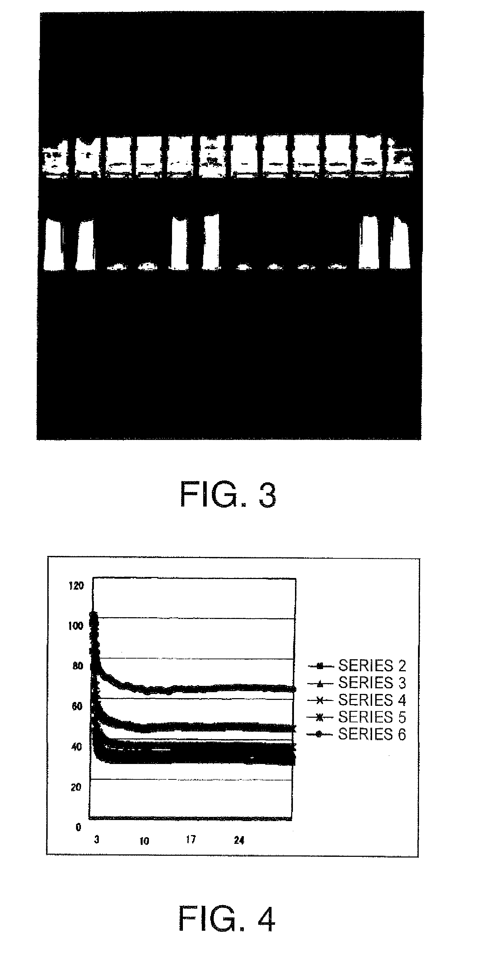

The results were as shown in Fig. 3. The drawing is an image taken 10 min after the start of monitoring.
In the drawing, twin pairs from the right respectively mean normal malt 1, premature yeast flocculating malt 1, premature yeast flocculating malt 2, normal malt 2, premature yeast flocculating malt 3, and normal malt 3.
Further, the numerical values of the degree of whiteness obtained by an image analysis of the image data shown in the drawing are shown in Table 1 below.

**[Table 1]**

| From left of Fig. 3 | No. 1 | No. 2 | No. 3 | No. 4 | No. 5 | No. 6 |
|---|---|---|---|---|---|---|
| Degree of whiteness | 81.1 | 78 | 24.8 | 35.2 | 83.7 | 83.5 |
| From left of Fig. 3 | No. 7 | No. 8 | No. 9 | No. 10 | No. 11 | No. 12 |
| Degree of whiteness | 27.3 | 30.1 | 24.8 | 24.7 | 84.5 | 79.5 |

### Example 2: Studies on measuring time

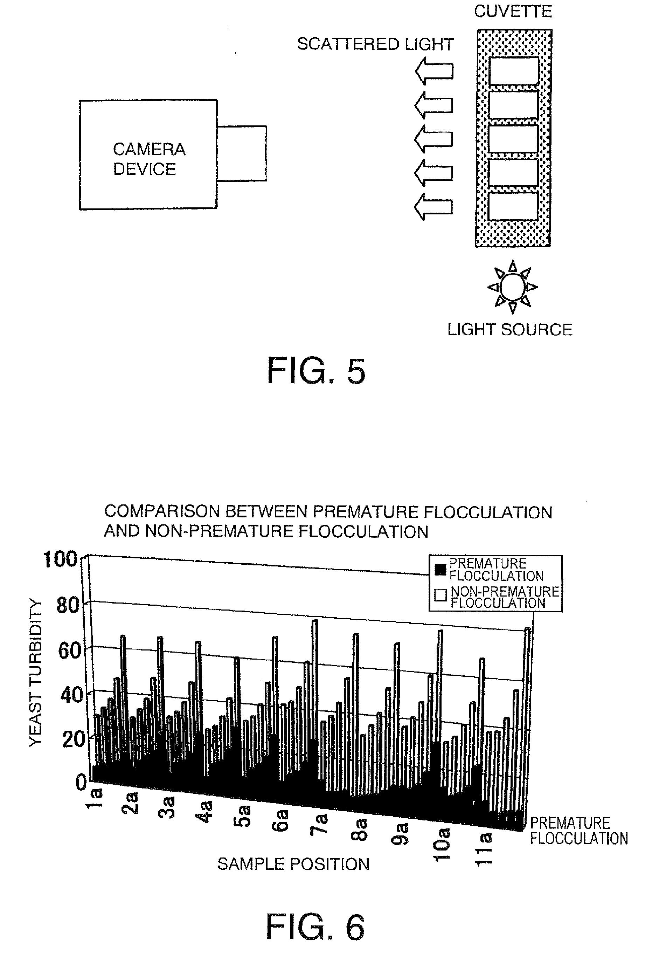

Samples of premature yeast flocculating malt 1 were provided in the same manner as in Example 1. According to Example 1, vials were considered as five divided stages (Fig. 5), each stage was monitored, and the degree of whiteness was measured over time from time 0 to time 30 min.

The results were as shown in Fig. 4. In the drawing, series 2 to 6 represent the results of measurement through windows divided into five stages, and the series 2 to 6 respectively mean the results measured at windows from the bottom to the top in that order. As a result of studies on the measuring time, it was found that, in all the stages, the turbidity was significantly decreased until the first 3 min had elapsed but, after that, remained relatively stable until 30 min had elapsed.

### Example 3: Studies on measuring position

Samples of the premature yeast flocculating malt and the non-premature yeast flocculating malt were provided in the same manner as in Example 1. Next, in order to examine the position of the vial or the cuvette for comparison which provides the highest accuracy, the vials were considered as five divided stages (Fig. 5), and the turbidity was compared for areas at the respective stages.

The results were as shown in Fig. 6. Fig. 6 shows data on the turbidity four min after the start of standing. For all the vials, the premature yeast flocculating malt and the non-premature yeast flocculating malt could be determined by measurement in areas at respective five divided stages.

### Example 4: Studies on buffer solution

In the same manner as in the item of "1) Cultivation of yeast" in the "Experimental method," yeast was cultivated, and cultivated yeast at a late stationary phase in the prepared growth curve was collected as follows and was used in the following experiment:
yeast 1: yeast 7 days after the start of cultivation; and
yeast 2: yeast 6 days, 16 hr after the start of cultivation.
   Regarding control, cultivated yeast at an early stationary phase (yeast 5 days, 10 hr after the start of cultivation) was used as "normal yeast" in the following experiment.
   Water was added to each of the cultivated yeast samples to a predetermined concentration (OD600: about 200) to give yeast liquids.

In the same manner as in the item of "3) Preparation of water-extracted high molecular fraction of malt in the "Experimental method," "normal malt 1" and "premature yeast flocculating malt 1" were provided as malt samples.

The same test as in "Example 1" was carried out to determined the degree of whiteness for each case, except that the yeast liquids provided above were used, the "normal malt 1" and the "premature yeast flocculating malt 1" were used as the water-extracted high molecular fraction of malt, and buffer solutions specified below were used.

The results were as shown in Table 2.

**[Table 2]**

| | | Conventional yeast | | Yeast 1 | | Yeast 2 | |
|---|---|---|---|---|---|---|---|
| Amount of component added acetate buffer-CaCl₂ (% by weigh) | saccharide to | Normal malt 1 | Premature yeast floccu-lating malt 1 | Normal malt 1 | Premat ure yeast floccu-I ating malt 1 | Normal malt 1 | Prema ture yeast flocculating malt 1 |
| Degree of whiteness | 0 (Not added) | 62.3 | 26.8 | 49.8 | 14.1 | 33.1 | 16.5 |
| | 1% Maltose | 92.7 | 92.5 | 86.6 | 45 | 76 | 37.5 |
| | 2% Maltose | 96.9 | 96.4 | 91.9 | 89 | 67.1 | 76.7 |
| | 3% Maltose | 97.4 | 98 | 93.3 | 94.7 | 92.6 | 93.2 |

As is apparent from the results of the case where no saccharide component was added, the use of highly flocculable yeasts (that is, yeast 1 and yeast 2) resulted in lowered degree of whiteness due to the flocculability of the yeast per se even when normal malt was used.
When highly flocculable yeasts were used, the addition of 1% maltose to the buffer solution developed a distinct difference in the degree of whiteness between the normal malt and the premature yeast flocculating malt. The development of the distinct difference demonstrates that whether or not malt used is premature yeast flocculable can be determined. In the above table, the difference was significant when the saccharide component used and the concentration were maltose and 1% by weight, respectively.

## Claims

1. A method for rapidly measuring an premature yeast flocculating factor of malt contained in a brewing material, comprising the following steps of:
(1) mixing yeast in the late logarithmic growth phase or thereafter with a water-extracted high molecular fraction prepared from a test material sample in a buffer solution to suspend the mixture of the yeast and the water-extracted high molecular fraction in the buffer solution; and
(2) irradiating the suspension obtained in the step (1) with visible light, photographing the scattered light with a camera device, image-analyzing the image data thus obtained and determining the degree of whiteness of the suspension to measure the degree of sedimentation of the yeast in the suspension.

2. The method according to claim 1, wherein, in the step (2), the scattered light is photographed with a digital camera device, and the image data thus obtained are image-analyzed to determine the digitized degree of whiteness of the suspension.

3. The method according to claim 1 or 2, wherein the degree of whiteness of the suspension is digitized by presuming the degree of whiteness at lights-outat lights-out and the degree of whiteness of the suspension in which the yeast immediately after the mixing and suspending is homogeneously dispersed to be 0 and 100, respectively.

4. The method according to any one of claims 1 to 3, wherein, in the step (2), the irradiation of the suspension with the visible light is carried out from a light source placed just under the suspension, and the light scattered in the suspension is photographed with a camera device installed in a direction horizontal to the suspension.

5. The method according to any one of claims 1 to 4, wherein, in the step (1), a plurality of analytes are subjected to simultaneous mixing and suspending in a multianalyte shaking device which allows a plurality of analytes to undergo simultaneous shaking, thereby obtaining a plurality of suspensions.

6. The method according to claim 5, wherein the degrees of sedimentation of yeasts in a plurality of respective suspensions are simultaneously measured.

7. The method according to any one of claims 1 to 6, wherein the yeast used in the step (1) is one obtained by cultivating yeast and harvesting yeast in the late logarithmic growth phase or thereafter, or one obtained by further cryopreserving the harvested yeast.

8. The method according to any one of claims 1 to 7, wherein the high molecular fraction used in the step (1) is a high molecular fraction prepared by subjecting the water extract of the test material sample to ethanol precipitation, or a high molecular fraction obtained by separating the water extract of the test material sample by dialysis, ultrafiltration, or gel filtration.

9. The method according to any one of claims 1 to 8, wherein the high molecular fraction used in the step (1) is prepared from a saccharified liquid of the test material sample.

10. The method according to any one of claims 1 to 9, wherein, in preparing the water-extracted high molecular fraction used in the step (1), the test material sample is enzymatically treated during the extraction.

11. The method according to any one of claims 1 to 10, wherein the buffer solution used in the step (1) is acetate buffer-CaCl₂.

12. The method according to any one of claims 1 to 10, wherein the buffer solution used in the step (1) is acetate boffer-CaCl₂ to which a saccharide component selected from the group consisting of glucose, maltose, mannose, and their mixtures has been added.

13. The method according to any one of claims 1 to 12, wherein the test material sample is barley, malt, or barley in the course of malting.

14. The method according to any one of claims 1 to 13, wherein the water-extracted high molecular fraction in the test material sample is a high molecular fraction of an extract obtained by extracting a ground product of malt with water for 30 sec or more, or a high molecular fraction of an extract obtained by extracting a ground product of barley or a ground product of barley in the course of malting with water for 15 min or more.

15. A method for rapidly determining an premature yeast flocculating property of malt in a brewing material, comprising the step of using a method according to any one of claims 1 to 14.

16. A process for producing malt, comprising the step of determining an premature yeast flocculating property of a malt raw material, in-process malt, or produced malt by a method according to any one of claims 1 to 14 to manage a malt production process.

17. A process for producing a fermentative alcohol beverage, comprising the step of determining an premature yeast flocculating property of a brewing material by a method according to any one of claims 1 to 14 to select and regulate the brewing material used.

18. An apparatus for continuously and quantitatively measuring the sedimentation of cells in a suspension, the apparatus comprising:
analyte shaking means that horizontally holds a plurality of cuvettes or vials, in which a suspension as an analyte is to be placed, at a predetermined position in a line, and if necessary shakes the cuvettes or vials to suspend the suspension contained in the cuvettes or vials;
a camera device that photographs the cuvettes or vials disposed in a line; and
data processing means that subjects image data photographed with the camera device to an image analysis to obtain digitized data on the degree of whiteness of the suspensions.

19. The apparatus according to claim 18, wherein the analyte shaking means has a visible light source just under the cuvettes or vials.

20. The apparatus according to claim 18 or 19, which further comprises control means that controls the analyte shaking means and the camera device so that, after shaking of the cuvettes or vials for a predetermined period of time in the analyte shaking means, the cuvettes or vials are allowed to stand for a predetermined period of time and are photographed with the camera device.

21. The apparatus according to any one of claims 18 to 20, wherein the camera device is a digital video camera.

22. The apparatus according to any one of claims 18 to 21, for use in rapid measurement of an premature yeast flocculating factor of malt contained in a brewing material.

23. The apparatus according to any one of claims 18 to 22, for use in carrying out a method according to any one of claims 1 to 14.
